# EUROPEAN PATENT SPECIFICATION

(11) **EP 3 395 400 B1**
(45) Date of publication and mention of the grant of the patent: **24.06.2020**
(21) Application number: 16877840.5
(22) Date of filing: 22.12.2016
(51) Int. Cl.: A61N 1/32, H02J 3/38, H02J 7/00

(54) **ELECTRONIC SYSTEM HAVING VARIABLE MODULAR POWER FOR GENERATING ELECTRICAL PULSES AND ASSOCIATED USES**
ELEKTRONISCHES SYSTEM MIT VARIABLER MODULARER LEISTUNG ZUR ERZEUGUNG VON ELEKTRISCHEN IMPULSEN UND ZUGEHÖRIGE VERWENDUNGEN
SYSTÈME ÉLECTRONIQUE DE PUISSANCE MODULAIRE VARIABLE POUR LA GÉNÉRATION D'IMPULSIONS ÉLECTRIQUES ET UTILISATIONS ASSOCIÉES

(30) Priority: 22.12.2015 ES 201531870
(43) Date of publication of application: 31.10.2018
(60) Divisional application: 20159887.7
(73) Proprietor: Universidad De Zaragoza, 50009 Zaragoza (ES); Universitat Pompeu Fabra, 08080 Barcelona (ES)
(72) Inventor: SARNAGO ANDÍA, Héctor, 50009 Zaragoza (ES); LUCÍA GIL, Óscar, 50009 Zaragoza (ES); BURDÍO PINILLA, José Miguel, 50009 Zaragoza (ES); NAVAL PALLARÉS, Alejandro, 50009 Zaragoza (ES); IVORRA CANO, Antoni, 08080 Barcelona (ES); CASTELLVÍ FERNÁNDEZ, Quim, 08080 Barcelona (ES)
(74) Representative: Hoffmann Eitle
(86) International application number: PCT/ES2016/070926
(87) International publication number: WO 2017/109261

(56) References cited:
- US-A1- 2007 242 743
- US-A1- 2011 065 161
- US-A1- 2015 155 716
- US-A1- 2015 263 526
- VARMA R ET AL.: 'Development of a solid state versatile pulsar for high voltage and high power applications' PULSED POWER CONFERENCE, 2009. PPC &APOS;09 28 June 2009, PISCATAWAY, NJ, USA, pages 1312 - 1316, XP031615086
- BERNAL CARLOS ET AL.: 'A review of pulse generation topologies for clinical electroporation' IECON 2015 - 41ST ANNUAL CONFERENCE OF THE IEEE INDUSTRIAL ELECTRONICS SOCIETY 09 November 2015, pages 625 - 630, XP032855334
- SUNGWOO BAE ET AL.: 'High- Power Pulse Generator With Flexible Output Pattern' IEEE TRANSACTIONS ON POWER ELECTRONICS vol. 25, no. 7, 01 July 2010, USA, ISSN 0885-8993 pages 1675 - 1684, XP011300088

## Description

### FIELD OF THE INVENTION

The present invention is comprised in the technical field of electroporation devices intended for use in medical treatments for improving the absorption of medicinal products or the destruction of tumor cells. More specifically, the invention relates to a modular, variable electronic power system for generating unipolar and bipolar electrical pulses. The sector of greatest interest of the invention is the biomedicine sector, although it is also applicable in other sectors, such as sterilization in the food industry.

### BACKGROUND OF THE INVENTION

Electroporation or electropermeabilization is a technique used in medicine which consists of applying a pulsed electric field to a living organism such that it experiences, at the cell membrane level, changes which may be temporary or permanent depending on the intensity of the applied field.

Said electrical pulses are produced by generators having different characteristics which vary depending on the electroporation technique to be used or on the problem to be solved. When the voltage going through a plasma membrane exceeds its dielectric rigidity, pores, which can close after a certain period of time, are formed. In the cases in which the opening of the pores is temporary and reversible, extracellular compounds may be introduced into the cell for therapeutic purposes. Alternatively, the pores can remain open irreversibly, giving rise to apoptotic cell death. In this context, the reversibility of the technique, as well as the size of the pores and the duration they remain open depend on the intensity of the applied electric field and on the time the cell is exposed to said field.

Irreversible electroporation (or "IRE") is a non-thermal ablation technique that is gaining a lot of interest today for the treatment of certain types of tumor with increased resistance. It consists of applying strong electric fields for the purpose of causing permeabilization of the cell membranes of the tissue to cause cell death. Some advantages of this technique with respect to conventional tumor ablation techniques are the possibility of treating regions close to large blood vessels since the thermal cooling will not affect them, or of preserving the connective tissue, blood vessels, and other ducts. To achieve irreversibility in the electroporation technique, the generator must reach a high voltage and current level, the threshold of which varies depending on the type of cells to be treated.

Unipolar pulse generators intended for medical applications today have insufficient maximum voltage levels for effective, widespread use in electroporation techniques. This is the case, for example, of the system disclosed in Review of Scientific Instruments 78, 034702 (2007), in the scientific paper entitled "Analysis of a modular generator for high-voltage, high-frequency pulsed applications, using low voltage semiconductors (1 kV) and series connected step-up (1:10) transformers" (L.M. Redondo *et al*.), which describes a modular generator that produces unipolar pulses which, despite being high voltage, are still below the voltage levels required for the applications described herein. Another similar case is the system disclosed in patent application WO 2011/017802 A1 (S. Jayaram et al.) which describes an electric generator having a plurality of modules connected in cascade and producing unipolar pulses having a variable output voltage, depending on the number of modules that are included in the system.

In this manner, even though the known modular generators allow solving some deficiencies of conventional techniques, they are limited in terms of the maximum voltages and currents that they can reach, and they also have severe constraints as regards the duration and configurability of the generated pulses, making the application thereof in the field of irreversible electroporation of tumor tissues difficult.

Likewise, existing generators for application thereof in irreversible electroporation which are capable of offering the required output voltages and currents are, however, rather non-versatile, where they allow obtaining a limited range of voltages and their use is likewise limited to certain specific types of cells or situations.

According to what has been described in the preceding paragraphs, there is a need in the present technical field for alternatives that allow solving the described problems for the purpose of attaining output voltage and current values suitable for widespread use in irreversible electroporation, as well as device versatility that allows adapting said device to a wide variety of medical situations or applications.

The present invention is intended to solve said problems by means of a novel modular system for generating high-voltage unipolar or bipolar electrical pulses.

### BRIEF DESCRIPTION OF THE INVENTION

An object of the present invention is therefore to provide a pulse generator technology based on a modular structure and unipolar or bipolar pulses, which allows greater versatility and higher output voltage than generators of the state of the art. To that end, the invention proposes a high-voltage generator based on a modular, versatile electronic power system which includes a control unit and allows adapting the intensity and other characteristics of the electrical pulses to each specific application, depending on the number of modules there are. Said generator is preferably applied in electroporation, where it can be adapted to different specific problems or organs given the versatility of the modular system and the capacity to achieve high voltage and current levels.

The mentioned object of the invention is preferably carried out by means of a system comprising:
- one or more electrical pulse generation modules, wherein said modules are connectable in series or in parallel. By means of the connection in series, the output voltage of said pulses is the sum of the individual output voltages of each module. By means of the connection in parallel, the total current is the sum of the currents of each module.
- a charging unit for the generation modules;
- a control unit for the generation modules and the charging unit.

Advantageously, the generation modules are coupled to the charging unit by means of isolation transformers, said charging unit being arranged as the primary of the transformers, and the generation modules as the secondaries of the transformers.

Likewise, each generation module preferably comprises an AC/DC rectifier connected to the output of its respective transformer, and a DC/AC inverter connected to said AC/DC rectifier in a bridge configuration for generating electrical output pulses or pulse trains; and the charging unit comprises a DC/DC step-up converter connected to an indirect DC/AC inverter, wherein said DC/AC inverter is connected to the input of the primary of the transformer.

Higher voltage and intensity in the pulses are obtained due to their bipolar nature and to the possibility of adding modules both in series and in parallel to the architecture of the device, which in turn raises a technical solution that adds versatility to the devices. Bipolar pulses are also achieved in the present invention as a result of the bridge configuration of the inverter inside each generation module.

More specifically, the generator of the invention allows obtaining pulses with a high voltage level (of the order of 10-15 kV peak to peak) and a high current level (400-600 A peak to peak), well exceeding generators that are available today in the clinical setting, and obtaining, in medical applications, more than twice the voltage and more than five times the current obtained with the technologies existing on the market. This means that with the generator of the invention, it is possible to achieve ablation volumes that are much greater than the volumes which can be achieved today, and by not using a low-frequency transformer, a more compact and lightweight solution compared to the solution offered by current generators is provided.

On the other hand, the modular design proposed by the system of the invention allows using the number of modules needed to achieve the voltage required in a given application. A greater versatility in the output voltage is thereby obtained by means of unipolar or bipolar pulses and pulse trains, having a width (from 1 µs) and number of pulses that are completely configurable. This configurability leads to the following technical advantages:
- Attenuating the effect of electrochemical reactions. These reactions are harmful both to the electrodes and to the organic tissues.
- Eliminating the formation of hydrogen and oxygen bubbles by hydrolysis.
- Less neurostimulation leading to unwanted muscle activation.
- Possibility of applying rapid bursts of short pulses, which significantly reduces total treatment time.

As an additional advantage, the system of the invention does not require using a transformer at the output. This constitutes a key difference as it obtains an output impedance that is much lower, and therefore less affected by the charge. This aspect is very important in electroporation as both the electrodes and the tissue to be connected are highly variable in terms of charge. The invention therefore allows assuring a square wave voltage form in the output at all times.

In a preferred embodiment of the invention, one or more pulse generation modules comprise an auxiliary AC/DC block, powered by the output of the isolation transformer, and likewise connected to the AC/DC rectifier and to the DC/AC inverter so as to generate a voltage for feeding same. In turn and in a similar manner, the charging unit preferably comprises an auxiliary DC/DC block, connected to the DC/DC step-up converter and to the indirect DC/AC inverter so as to generate a voltage for feeding same.

In another preferred embodiment of the invention, the frequency of the indirect DC/AC inverter of the charging unit is equal to or greater than 200 kHz, and the isolation voltage of the transformers is preferably equal to or greater than 15 kV.

In another preferred embodiment of the invention, the generator includes a control architecture based on a programmable logic device (FPGA) which allows the current and future implementation of advanced synchronization functions with ECG, protections, treatment automation, etc. A greater degree of versatility and adaptation of the pulses of the output voltage to the treatment to be performed is thereby achieved.

The control unit of the system of the invention likewise provides the capacity to program the number of active generation modules while applying the pulses. This allows quickly varying the magnitude of the applied pulses or pulse trains, thereby configuring the form thereof (for example, it is possible to apply pulses or pulse trains in the form of a step). This capacity is of interest, for example, in applications relating to electroporation-assisted gene transfection (or "gene electrotransfer"). It has been demonstrated in this field of application of electroporation that protocols consisting of a high-magnitude short pulse, followed by another low-magnitude long pulse, are more effective than two or more high-magnitude short pulses.

In another preferred embodiment of the invention, the control unit comprises at least one connection with the generation modules and at least one connection with the charging unit, said connections being isolated by means of optical fibers. Improved isolation which increases safety when using the system is thereby achieved.

In another preferred embodiment of the invention, the generator is powered by means of batteries, and not through direct connection to the power grid like in generators used today, thereby improving safety and isolation during use, and facilitating the standardization process and compliance with electromagnetic compatibility regulations.

In another preferred embodiment of the invention, the generator comprises a wireless communication subsystem for communication by means of a WiFi connection with a computer, through which various parameters such as polarity, amplitude, the number of pulses in each burst, the number of bursts, and their frequency of repetition, can be configured. This possibility of wireless control significantly increases safety and ease of application.

Another object of the present invention relates to the associated uses of the system, comprising applications for food sterilization, waste treatment, contamination control, metal or semiconductor treatment, molecular biology assays, and/or medical or cosmetic treatments. The uses of the system associated with molecular biology assays and medical and/or cosmetic treatments preferably comprise the applications of electroporation.

### DESCRIPTION OF THE DRAWINGS

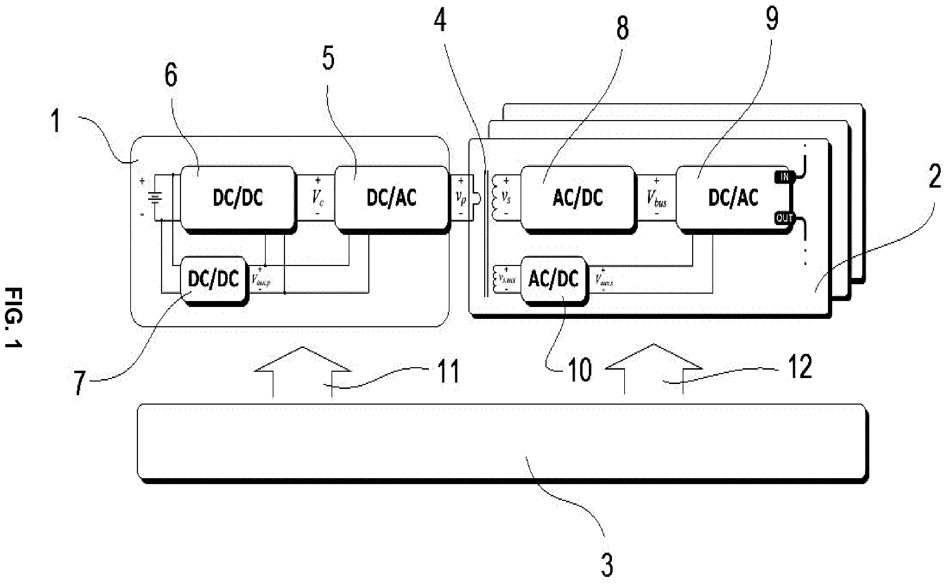

Figure 1 shows a block diagram of the modular, versatile electronic power system of the invention according to a preferred embodiment thereof.

### DETAILED DESCRIPTION OF THE INVENTION

A detailed description of the invention is provided below in reference to a preferred embodiment thereof based on Figure 1 herein. Said embodiment is provided to illustrate and not limit the claimed invention.

As described in the preceding sections, the high-voltage generator proposed by the present invention is based on a modular, versatile electronic power system which allows adapting the design to the output voltage and to the characteristics of the required pulses, depending on the specific application or treatment to be applied.

Said Figure 1 shows the general block diagram of the modular electronic power system of the invention, where said system essentially comprises a charging unit (1), one or more pulse generation modules (2) in a rectifier-inverter configuration, and a control unit (3). The pulse generation modules (2) are connected to the charging unit (1) by means of magnetic coupling through an isolation transformer (4).

The charging unit (1) is preferably formed by a high-frequency indirect DC/AC inverter (5), which is connected to an upstream DC/DC step-up converter (6). The main function of said charging unit (1) is to charge each of the generation modules (2) to the required voltage by means of the isolation transformer (4) by means of which said charging unit (1) and said generation modules (2) are coupled. It is important to highlight that the required isolation (greater than 15 kV) is achieved as a result of the coupling by means of the transformer (4), and a compact implementation of the system is achieved due to the high operating frequency (typically 200 kHz),.

In addition to the preceding elements, the charging unit (1) comprises an auxiliary DC/DC block (7) which is in charge of delivering a supply voltage V_{aux,p} for controlling the DC/AC inverter (5) and the DC/DC step-up converter (6).

In turn, the pulse generation modules (2), arranged in the secondary of the isolation transformer (4) of the system are in charge of generating the output voltage that will be applied during electroporation treatment. Each module (2) preferably consists of an AC/DC rectifier (8) and a DC/AC inverter (9), based on a bridge configuration, so as to have the capacity to generate bipolar output voltage pulses in each module (2).

Like the arrangement of the elements of the charging unit (1), each pulse generation module (2) can comprise an auxiliary AC/DC block (10), also powered from the secondary of the isolation transformer (4), which is in charge of generating the supply voltages V_{aux,s} of the AC/DC rectifier (8) and the DC/AC inverter (9).

The pulse generation modules (2) of the system of the invention can be interconnected in series, giving rise to an output voltage the value of which will be the sum of the voltages generated by each of the generation modules (2) separately. Likewise, the modules can be connected in parallel, so the delivered current will be the sum of the currents of each module. The invention thereby provides a variable power stage which allows being adapted to the needs of the treatment to be performed so as to generate the required voltage and current levels.

As described above, the system of the invention also comprises a control unit (3) which is in charge of controlling the electronic power system formed by the charging unit (1) and each of the pulse generation modules (2). The control signals of the generation modules (2) are preferably emitted using a programmable logic device (FPGA) integrated in said control unit (3). It is worth noting that the generation of the control signals by means of FPGA allows a greater degree of versatility and adaptation of the output voltage pulses to the treatment to be performed. This is not possible in current commercial systems which have severe constraints in terms of the types of voltage pulses they are capable of generating.

As mentioned in the preceding sections, the control unit (3) is preferably configured with programming means for programming the number of active generation modules (2) of the system while applying the pulses, which allows quickly varying the magnitude of the applied pulses or pulse trains, configuring the form thereof.

In a complementary manner, and due to the strict isolation requirements imposed by regulations governing the use and safety of electroporation devices, the control signals are preferably isolated by means of optical fibers (11, 12).

Finally, the system of the invention is preferably communicated through wireless means, for example by means of a WiFi network connected to a remote computer (not shown in Figure 1), through which the polarity, amplitude, the number of pulses in each burst, the number of bursts and their frequency of repetition, are configured.

The system of the invention provides satisfactory results both in the treatments of plant tissues and in the treatments of living animal tissues.

## Claims

1. A modular, variable electronic power system for generating unipolar or bipolar electrical pulses, comprising:
- one or more electrical pulse generation modules (2), wherein said modules (2) are connectable in series or in parallel, such that the output voltage and/or current of the pulses is respectively the sum of the individual output voltages and/or currents of each module (2);
- a charging unit (1) for powering the pulse generation modules (2); and
- a control unit (3) for controlling the generation modules (2) and the charging unit (1);
wherein the generation modules (2) are coupled to the charging unit (1) by means of an isolation transformer (4), said charging unit (1) being connected to the primary side of the transformer (4), and the generation modules (2) connected to a secondary side of the transformers (4);
the system **characterized in that**
- each generation module (2) comprises an AC/DC rectifier (8) connected to an output of the secondary side of the transformer (4), and a DC/AC inverter (9) having a bridge configuration
connected to the output of said AC/DC rectifier (8)for generating the electrical output pulses or pulse trains; and **in that**
- the charging unit (1) comprises a DC/DC converter (6) connected to an DC/AC inverter (5), wherein said DC/AC inverter (5) is connected to the primary side of the transformer (4).

2. The system according to the preceding claim, wherein one or more generation modules (2) comprise an auxiliary AC/DC block (10), powered by the output of the isolation transformer (4), and also connected to the AC/DC rectifier (8) and to the DC/AC inverter (9) so as to generate a voltage for the feeding both.

3. The system according to any of the preceding claims, wherein the charging unit (1) comprises an auxiliary DC/DC block (7), connected to the DC/DC converter (6) and to the DC/AC inverter (5) so as to generate a voltage for feeding both.

4. The system according to any of the preceding claims, wherein the frequency of the DC/AC inverter (5) of the charging unit (1) is equal to or greater than 200 kHz.

5. The system according to any of the preceding claims, wherein the isolation voltage of the transformer (4) is equal to or greater than 15 kV.

6. The system according to any of the preceding claims, wherein the control unit (3) is programmable to control the activation of the generation modules (2) while generating the pulses, for varying the magnitude of the applied pulses or pulse trains.

7. The system according to any of the preceding claims, wherein the control unit (3) comprises at least one connection with the generation modules (2) and at least one connection with the charging unit (1), said connections being isolated by means of optical fibers (11,12).

8. The system according to any of the preceding claims, wherein the control unit (3) is configured with programming means for programming the number of active generation modules (2) of the system during application of the pulses.

9. The system according to any of the preceding claims, comprising batteries for powering the charging unit (1), the generation modules (2), and the control unit (3).

10. The system according to the preceding claims, comprising at least one communication subsystem for communication with a computer, for the configuration of polarity, amplitude, number of pulses or pulse trains, and/or the frequency of repetition thereof.

11. The system according to the preceding claim, wherein the communication subsystem comprises a wireless connection with the computer through WiFi.

12. The system according to any of the preceding claims for use in applications for food sterilization, waste treatment, contamination control, metal or semiconductor treatment, molecular biology assays, and/or medical or cosmetic treatments.

13. The system according to any of the preceding claims for use in electroporation techniques.

## Patentansprüche

1. Modulares System mit variabler elektronischer Leistung zur Erzeugung von unipolaren oder bipolaren elektrischen Impulsen, umfassend:
- ein oder mehrere elektrische Impulserzeugungsmodule (2), wobei die genannten Module (2) in Reihe oder parallel verbindbar sind, so dass die Ausgangsspannung und/oder der Strom der Impulse jeweils die Summe der einzelnen Ausgangsspannungen und/oder Ströme aus jedem Modul (2) ist;
- eine Ladeeinheit (1) zur Versorgung der Impulserzeugungsmodule (2); und
- eine Steuereinheit (3) zur Steuerung der Erzeugungsmodule (2) und der Ladeeinheit (1);
wobei die Erzeugungsmodule (2) mit der Ladeeinheit (1) mittels eines Isoliertransformators (4) gekoppelt sind, wobei die genannte Ladeeinheit (1) mit der Primärseite des Transformators (4) verbunden ist, und die Erzeugungsmodule (2) mit einer Sekundärseite der Transformatoren (4) verbunden sind;
wobei das System **dadurch gekennzeichnet ist, dass**
- jedes Erzeugungsmodul (2) einen AC/DC-Gleichrichter (8), welcher mit einem Ausgang der Sekundärseite des Transformators (4) verbunden ist, und einen DC/AC-Wechselrichter (9) mit einer Brückenausbildung, welcher mit dem Ausgang des genannten AC/DC-Gleichrichter (8) zur Erzeugung von den elektrischen Ausgangsimpulsen oder Impulsfolgen verbunden ist, umfasst; und dass
- die Ladeeinheit (1) einen DC/DC-Wandler (6) umfasst, welcher mit einem DC/AC-Wechselrichter (5) verbunden ist, wobei der genannte DC/AC-Wechselrichter (5) mit der Primärseite des Transformators (4) verbunden ist.

2. System nach dem vorhergehenden Anspruch, wobei ein oder mehrere Erzeugungsmodule (2) einen AC/DC-Hilfsblock (10) umfassen, welcher vom Ausgang des Isoliertransformators (4) versorgt wird, und auch mit dem AC/DC-Gleichrichter (8) und mit dem DC/AC-Wechselrichter (9) verbunden ist, um eine Spannung zur Versorgung von Beiden zu erzeugen.

3. System nach einem der vorhergehenden Ansprüche, wobei die Ladeeinheit (1) einen DC/DC-Hilfsblock (7) umfasst, welcher mit dem DC/DC-Wandler (6) und mit dem DC/AC-Wechselrichter (5) verbunden ist, um eine Spannung zur Versorgung von Beiden zu erzeugen.

4. System nach einem der vorhergehenden Ansprüche, wobei die Frequenz des DC/AC-Wechselrichters (6) der Ladeeinheit (1) gleich oder größer als 200 kHz ist.

5. System nach einem der vorhergehenden Ansprüche, wobei die Isolationsspannung des Transformators (4) gleich oder größer als 15 kV ist.

6. System nach einem der vorhergehenden Ansprüche, wobei die Steuereinheit (3) programmierbar ist, um die Aktivierung der Erzeugungsmodule (2) während der Erzeugung der Impulse zu steuern, um die Größe der angelegten Impulse oder Impulsfolgen zu ändern.

7. System nach einem der vorhergehenden Ansprüche, wobei die Steuereinheit (3) mindestens eine Verbindung mit den Erzeugungsmodulen (2) und mindestens eine Verbindung mit der Ladeeinheit (1) umfasst, wobei die genannten Verbindungen mittels optischer Fasern (11, 12) isoliert sind.

8. System nach einem der vorhergehenden Ansprüche, wobei die Steuereinheit (3) mit Programmiermitteln zur Programmierung der Anzahl von aktiven Erzeugungsmodulen (2) des Systems während der Anlegung der Impulse ausgebildet ist.

9. System nach einem der vorhergehenden Ansprüche, umfassend Batterien zur Versorgung der Ladeeinheit (1), der Erzeugungsmodule (2) und der Steuereinheit (3).

10. System nach den vorhergehenden Ansprüchen, umfassend mindestens ein Kommunikationssubsystem zur Kommunikation mit einem Computer, für die Ausbildung der Polarität, Amplitude, Anzahl von Impulsen oder Impulsfolgen und/oder der Wiederholungsfrequenz derselben.

11. System nach dem vorhergehenden Anspruch, wobei das Kommunikationssubsystem eine drahtlose Verbindung mit dem Computer über WLAN umfasst.

12. System nach einem der vorhergehenden Ansprüche für dessen Verwendung in Anwendungen für die Lebensmittelsterilisation, die Abfallbehandlung, die Kontaminationskontrolle, die Behandlung von Metallen oder Halbleitern, Molekularbiologieversuche und/oder medizinische oder kosmetische Behandlungen.

13. System nach dem vorhergehenden Anspruch für dessen Verwendung in Elektroporationstechniken.

## Revendications

1. Système électronique de puissance modulaire variable pour la génération d'impulsions électriques unipolaires ou bipolaires, comprenant :
- un ou plusieurs modules de génération d'impulsions électriques (2), dans lequel lesdits modules (2) peuvent être connectés en série ou en parallèle de manière que la tension et/ou le courant de sortie des impulsions est respectivement l'addition des tensions et/ou des courants de sortie individuels de chaque module (2) ;
- une unité de chargement (1) pour alimenter les modules de génération d'impulsions (2) ; et
- une unité de commande (3) pour commander les modules de génération (2) et l'unité de chargement (1) ;
dans lequel les modules de génération (2) sont couplés à l'unité de chargement (1) par le biais d'un transformateur d'isolement (4), ladite unité de chargement (1) étant connectée au côté primaire du transformateur (4), et les modules de génération (2) étant connectés à un côté secondaire des transformateurs (4) ;
le système étant **caractérisé en ce que**
- chaque module de génération (2) comprend un redresseur CA/CC (8) connecté à une sortie du côté secondaire du transformateur (4), et un onduleur CC/CA (9) ayant un configuration de pont connectée à la sortie dudit redresseur CA/CC (8) pour la génération de impulsions ou de trains d'impulsions de sortie électriques ; et **en ce que**
- l'unité de chargement (1) comprend un convertisseur CC/CC (6) connecté à un onduleur CC/CA (5), dans lequel ledit onduleur CC/CA (5) est connecté au côté primaire du transformateur (4).

2. Système selon la revendication précédente, dans lequel un ou plusieurs modules de génération (2) comprennent un bloc CA/CC auxiliaire (10) alimenté par la sortie du transformateur d'isolement (4), et connecté également au redresseur CA/CC (8) et à l'onduleur CC/CA (9) de manière à générer une tension pour l'alimentation des deux.

3. Système selon l'une quelconque des revendications précédentes, dans lequel l'unité de chargement (1) comprend un bloc CC/CC auxiliaire (7), connecté au convertisseur CC/CC (6) et à l'onduleur CC/CA (5) de manière à générer une tension pour l'alimentation des deux.

4. Système selon l'une quelconque des revendications précédentes, dans lequel la fréquence de l'onduleur CC/CA (5) de l'unité de chargement (1) est égale ou supérieure à 200 kHz.

5. Système selon l'une quelconque des revendications précédentes, dans lequel la tension d'isolement du transformateur (4) est égale ou supérieure à 15 kV.

6. Système selon l'une quelconque des revendications précédentes, dans lequel l'unité de commande (3) est programmable pour commander l'activation des modules de génération (2) pendant la génération des impulsions, pour faire varier la magnitude des impulsions ou des trains d'impulsions appliqués.

7. Système selon l'une quelconque des revendications précédentes, dans lequel l'unité de commande (3) comprend au moins une connexion avec les modules de génération (2) et au moins une connexion avec l'unité de chargement (1), lesdites connexions étant isolées par le biais de fibres optiques (11, 12).

8. Système selon l'une quelconque des revendications précédentes, dans lequel l'unité de commande (3) est configurée avec des moyens de programmation pour programmer le nombre de modules de génération actifs (2) du système pendant l'application des impulsions.

9. Système selon l'une quelconque des revendications précédentes, comprenant des batteries pour alimenter l'unité de chargement (1), les modules de génération (2) et l'unité de commande (3).

10. Système selon les revendications précédentes, comprenant au moins un sous-système de communication pour la communication avec un ordinateur, pour la configuration de la polarité, l'amplitude, le nombre d'impulsions ou trains d'impulsions, et/ou la fréquence de répétition de ceux-ci.

11. Système selon la revendication précédente, dans lequel le sous-système de communication comprend une connexion sans-fil avec l'ordinateur à travers de WiFi.

12. Système selon l'une quelconque des revendications précédentes, pour son utilisation dans des applications pour la stérilisation d'aliments, le traitement de déchets, le contrôle de la contamination, le traitement de métaux ou semi-conducteurs, des essais de biologie moléculaire et/ou des traitements médicaux ou cosmétiques.

13. Système selon l'une quelconque des revendications précédentes, pour son utilisation dans des techniques d'électroporation.
